# EUROPEAN PATENT APPLICATION

(11) **EP 4 442 137 A1**
(43) Date of publication of application: **09.10.2024**
(21) Application number: 21966050.3
(22) Date of filing: 02.12.2021
(51) Int. Cl.: A24F 40/46, A24F 40/40

(54) **ATOMIZATION ASSEMBLY AND ELECTRONIC ATOMIZATION DEVICE**

(71) Applicant: Shenzhen Smoore Technology Limited, Shenzhen, Guangdong 518102 (CN)
(72) Inventor: WANG, Chengtao, Shenzhen, Guangdong 518102 (CN); YANG, Jiyong, Shenzhen, Guangdong 518102 (CN)
(74) Representative: Herrero & Asociados, S.L.
(86) International application number: PCT/CN2021/135137
(87) International publication number: WO 2023/097618

(57) **Abstract**

An atomization assembly (1) and an electronic atomization device. The atomization assembly (1) comprises a housing assembly (10) and a heating body (12); the housing assembly (10) is provided with a liquid storage cavity (13), an air outlet channel (14) and an atomization cavity (17); the liquid storage cavity (13) is used for storing a substrate; the heating body (12) is used for atomizing the substrate to generate an aerosol; an atomization surface of the heating body (12) is exposed to the atomization cavity (17) and is basically parallel to the air outlet channel (14); the atomization cavity (17) is in communication with the atomization surface of the heating body (12) and the air outlet channel (14). By means of the configurations, the air channel is shortened, and contact between the aerosol and the wall surface is reduced; thus, formation of condensate is reduced, the temperature of the aerosol reaching the user mouth is proper, and the taste can be improved.

## Description

### TECHNICAL FIELD

This application relates to the field of atomization assembly technologies, and in particular, to an atomization assembly and an electronic atomization device.

### BACKGROUND

An electronic atomization device generally includes an atomization assembly and a power supply assembly. The atomization assembly includes a liquid storage cartridge, an airflow channel, and an atomization core. The airflow channel includes an air inlet channel, an atomization chamber, and an air outlet channel. The power supply assembly includes a power supply and a control circuit. Liquid inside the liquid storage cartridge flows to the atomization core. When a user inhales, the control circuit controls the power supply to supply electric energy for the atomization core to heat and atomize the liquid to generate an aerosol. Air enters from the air inlet channel and carries the aerosol inside the atomization chamber out from the air outlet channel.

At present, atomization surfaces of most atomization cores face downward. When coming out from the atomization chamber and the air outlet channel, the aerosol passes through some detours, which increases the contact between the aerosol and the wall surface, resulting in an increased amount of formed condensate. In addition, the length of an air passage is also increased, resulting in a lower temperature of the aerosol arriving at an air outlet.

### SUMMARY

This application provides an atomization assembly and an electronic atomization device, to resolve the technical problem of how to reduce the length of an air passage and reduce the contact between an aerosol and the wall surface in the related art.

To resolve the foregoing technical problem, the first technical solution provided in this application is: An atomization assembly is provided, including a housing, a heating seat, and an atomization core. The housing includes a liquid storage chamber, an air outlet channel, and an accommodating chamber. The liquid storage chamber is configured to store a substance. The heating seat is located inside the accommodating chamber. The atomization core is located inside the heating seat. The atomization core is configured to atomize the substance. An atomization surface of the atomization core is arranged facing toward the sidewall of the housing. The atomization surface of the atomization core cooperates with the heating seat to form an atomization chamber. The atomization chamber communicates with the air outlet channel.

The atomization surface of the atomization core is substantially parallel to the central axis of the atomization assembly. The air outlet channel is entirely arranged substantially parallel to the central axis of the atomization assembly. In addition, one end of the air outlet channel extends to the end surface of the housing to form an inhalation opening, and the other end thereof communicates with the atomization chamber.

The heating seat is arranged with a liquid drain hole and/or a liquid drain channel communicating with each other. The liquid drain hole communicates with the liquid storage chamber, and the sidewall of the liquid drain channel is arranged with a liquid inlet hole, to enable the atomization core be in fluid communication with the liquid storage chamber. The atomization assembly further includes an isolation plug. The isolation plug includes a sealing part and a pull rod. The sealing part is arranged inside the liquid drain channel and/or the liquid drain hole. The pull rod is arranged at the end of the sealing part away from the liquid storage chamber. The length of the sealing part is not less than the height of the liquid drain hole.

The heating seat includes a first sub-heating seat and a second sub-heating seat that are fixedly connected, and the first sub-heating seat and the second sub-heating seat cooperate with each other to clamp the atomization core.

A protrusion is arranged on one of the first sub-heating seat and the second sub-heating seat includes, and a hook is arranged on the other. The protrusion is arranged in cooperation with the hook. The first sub-heating seat and the second sub-heating seat are snap-connected by the protrusion and the hook.

The first sub-heating seat or the second sub-heating seat is provided with a groove, and the orientation of the opening of the groove is perpendicular to the central axis of the atomization assembly. The groove communicates with the liquid storage chamber, and the atomization core is arranged inside the groove.

The atomization assembly further includes a sealing member, and the sealing member is arranged between the atomization core and the bottom wall of the groove. The middle part of the sealing member is provided with a communication hole, to expose at least a part of the atomization core.

The atomization assembly further includes a liquid guiding element, and the liquid guiding element is arranged between the atomization core and the bottom wall of the groove.

The liquid guiding element is a liquid guiding cotton or a porous ceramic.

The first sub-heating seat includes a top cover and a lower seat, and the second sub-heating seat includes an upper seat and a base. The top cover of the first sub-heating seat covers the base of the second sub-heating seat and abuts against the upper seat of the second sub-heating seat. The base of the second sub-heating seat covers the top cover of the first sub-heating seat and abuts against the lower seat of the first sub-heating seat. A side surface of the lower seat of the first sub-heating seat is provided with the groove.

The top cover of the first sub-heating seat is provided with a liquid drain hole and a ventilation hole. One end of the ventilation hole communicates with the atomization chamber, and the other end of the ventilation hole communicates with the air outlet channel. One end of the liquid drain hole communicates with the liquid storage chamber. The lower seat of the first sub-heating seat is arranged with a liquid drain channel, one end of the liquid drain channel communicates with the other end of the liquid drain hole, and the other end of the liquid drain channel communicates with the groove. The atomization surface of the atomization core cooperates with a side surface of the upper seat of the second sub-heating seat to form the atomization chamber. The base of the second sub-heating seat is provided with an air inlet hole, and the air inlet hole communicates with the atomization chamber.

The liquid drain channel runs through the lower seat of the first sub-heating seat. The bottom wall of the groove is arranged with a liquid inlet hole, to enable the groove to communicate with the liquid drain channel. The atomization assembly further includes an isolation plug, and the isolation plug is partially arranged inside the liquid drain channel and/or the liquid drain hole. When the isolation plug is located at a first position, the liquid storage chamber does not communicate with the groove, and when the isolation plug is located at a second position, the liquid storage chamber communicates with the groove.

The isolation plug includes a sealing part and a pull rod. The sealing part is arranged inside the liquid drain channel and/or the liquid drain hole, and the pull rod is arranged at the end of the sealing part away from the liquid storage chamber. The base of the second sub-heating seat is provided with a connection hole, and the connection hole is provided corresponding to the liquid drain channel. The pull rod is arranged inside the connection hole, to connect to the sealing part. The end part of the pull rod extends out of the side of the housing facing away from the inhalation opening.

The length of the sealing part is not less than the height of the liquid drain hole.

The atomization core includes a dense base and a heating film. The dense base includes a liquid-absorbing surface and an atomization surface opposite to the liquid-absorbing surface, the dense base is provided with a plurality of first micropores, the first micropores are through holes running through the liquid-absorbing surface and the atomization surface, and the first micropores are configured to guide the substance to the atomization surface. The heating film is arranged on the atomization surface.

The atomization core further includes electrodes, the electrodes are arranged on the atomization surface, and the heating film is electrically connected to the electrodes. The atomization assembly further includes an elastic piece, one end of the elastic piece is connected to the electrodes, and the other end of the elastic piece is connected to the power supply assembly. The base of the second sub-heating seat is provided with a mounting hole, and the other end of the elastic piece is arranged inside the mounting hole. The elastic piece and the second sub-heating seat are injection molded.

The dense base is glass, and the glass is borosilicate glass, quartz glass, or photosensitive lithium aluminosilicate glass.

The thickness of the dense base ranges from 0.1 mm to 1 mm. The pore size of the first micropore ranges from 1 µm to 100 µm.

The first sub-heating seat and the second sub-heating seat are both integrally formed. To resolve the foregoing technical problems, the second technical solution provided in this application is: An electronic atomization device is provided, including an atomization assembly and a power supply assembly. The atomization assembly is the atomization assembly of any one of the foregoing aspects. The power supply assembly controls the atomization assembly to work.

Technical effects of this application: Different from the related art, the atomization assembly of this application includes a housing assembly and an atomization core. The housing assembly includes a liquid storage chamber, an air outlet channel, and an atomization chamber. The liquid storage chamber is configured to store a substance. The atomization core is configured to atomize the substance to generate an aerosol. An atomization surface of the atomization core is exposed to the atomization chamber and is substantially parallel to the air outlet channel, and the atomization chamber communicates with the atomization surface of the atomization core and the air outlet channel. Through the foregoing arrangement, the length of the air passage is reduced, and the contact between the aerosol and the wall surface is reduced, thereby reducing the amount of formed condensate and making the temperature of the aerosol arriving at the mouth of a user appropriate, which is beneficial to improving the taste.

### BRIEF DESCRIPTION OF THE DRAWINGS

To describe the technical solutions of the embodiments of this application more clearly, the following briefly introduces the accompanying drawings required for describing the embodiments. Apparently, the accompanying drawings in the following description show merely some embodiments of this application, and a person of ordinary skill in the art may still derive other drawings from the accompanying drawings without creative efforts.
FIG. 1 is a schematic structural diagram of an electronic atomization device according to this application.
FIG. 2 is a schematic structural diagram of an atomization assembly according to this application.
FIG. 3 is a schematic partial exploded structural view of the atomization assembly shown in FIG. 2.
FIG. 4 is a schematic structural diagram of an atomization core shown in FIG. 2.
FIG. 5 is a schematic structural diagram of a dense base shown in FIG. 4.
FIG. 6 is a schematic structural diagram of a first sub-heating seat shown in FIG. 2.
FIG. 7 is a schematic structural diagram of the first sub-heating seat shown in FIG. 6 from another perspective.
FIG. 8 is a schematic structural diagram of a second sub-heating seat shown in FIG. 2.
FIG. 9 is a schematic structural diagram of the second sub-heating seat shown in FIG. 8 from another perspective.
FIG. 10 is a partially enlarged view of some elements of the atomization assembly shown in FIG. 3.

### DETAILED DESCRIPTION

This application is further described below in detail with reference to the accompanying drawings and embodiments. It should be specifically noted that, the following embodiments are merely used for describing this application rather than limiting the scope of this application. Similarly, the following embodiments are merely some rather than all of the embodiments of this application, and all other embodiments obtained by a person of ordinary skill in the art without creative efforts shall fall within the protection scope of this application.

The terms "first", "second", and "third" in this application are merely intended for a purpose of description, and shall not be understood as an indication or implication of relative importance or implicit indication of the number of indicated technical features. Therefore, features defined with "first", "second", and "third" can explicitly or implicitly include at least one of the features. In the description of this application, "a plurality of" means at least two, such as two and three unless it is specifically defined otherwise. All directional indications (for example, up, down, left, right, front, back...) in the embodiments of the present disclosure are only used for explaining relative position relationships, movement situations or the like between the various components in a specific posture (as shown in the accompanying drawings). If the specific posture changes, the directional indications change accordingly. In the embodiments of this application, the terms "include", "have", and any variant thereof are intended to cover a non-exclusive inclusion. For example, a process, a method, a system, a product, or a device that includes a series of steps or units is not limited to the listed steps or units, but optionally further includes unlisted steps or units, or optionally further includes other inherent steps or components of the process, the method, the product, or the device.

An "embodiment" mentioned in this specification means that a particular feature, structure, or characteristic described with reference to this embodiment may be included in at least one embodiment of this application. The phrase appear at various positions in this specification may neither necessarily mean a same embodiment, nor mean an independent or optional embodiment exclusive from another embodiment. It is explicitly and implicitly understood by a person skilled in the art that embodiments described in the specification may be combined with another embodiment.

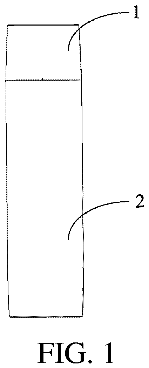

Referring to FIG. 1, FIG. 1 is a schematic structural diagram of an electronic atomization device according to this application. The electronic atomization device is configured to atomize a substance. The electronic atomization device includes an atomization assembly 1 and a power supply assembly 2 that are connected to each other. The atomization assembly 1 is configured to store the substance and atomize the substance to form an aerosol. The substance is generally liquid, alternatively, it may be solid or a solid-liquid mixture. The liquid substance may be a liquid substance such as a medicinal liquid or a plant grass liquid, etc. The atomization assembly 1 may be specifically used in different fields such as medical treatment, electronic aerosolization, etc. The power supply assembly 2 includes a battery (not shown), an airflow sensor (not shown), a controller (not shown), and the like. The battery is configured to supply power to the atomization assembly 1, for the atomization assembly 1 to atomize the substance to form the aerosol. The airflow sensor is configured to detect an airflow change in the electronic atomization device. The controller is configured to control, according to the airflow change detected by the airflow sensor, whether the atomization assembly 1 works. The atomization assembly 1 and the power supply assembly 2 may be integrally arranged or detachably connected, which is designed according to specific requirements.

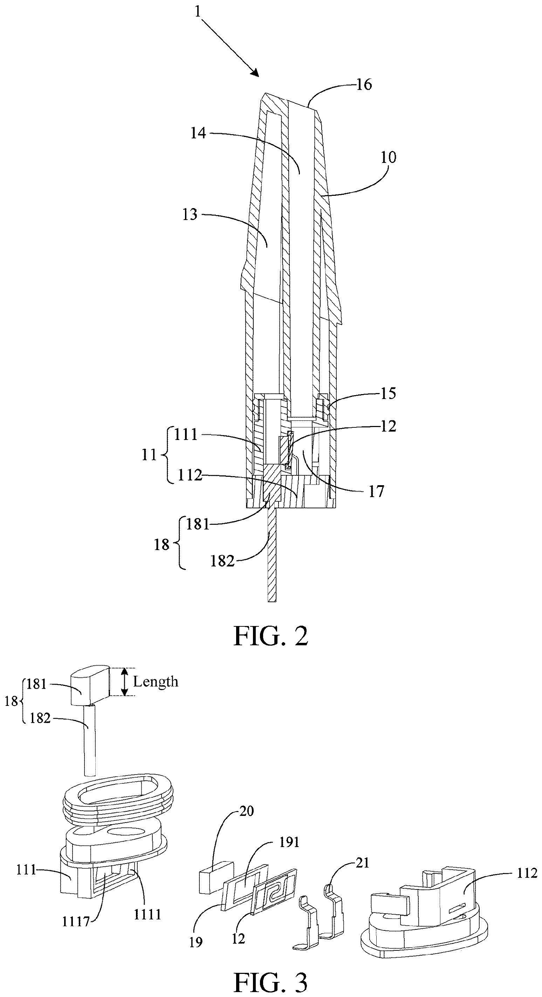

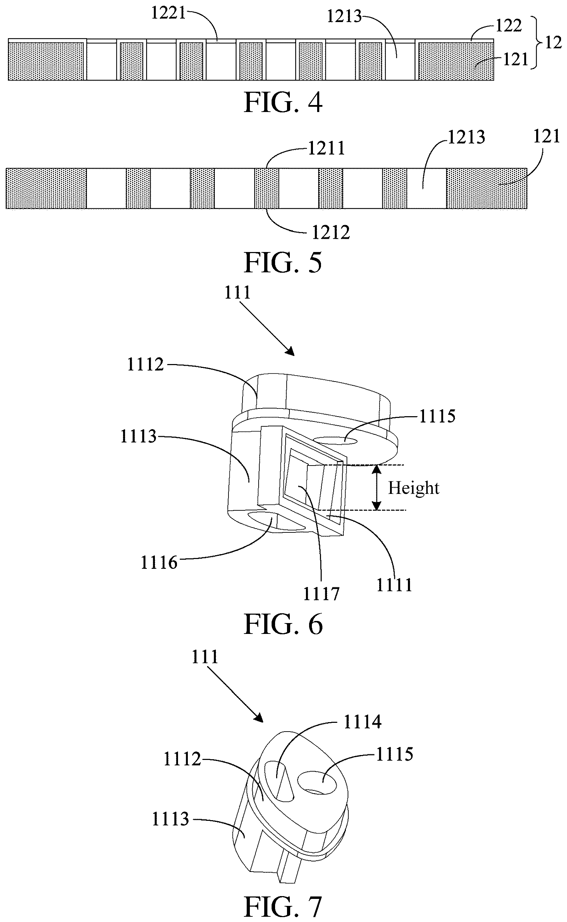

Referring to FIG. 2 to FIG. 5, FIG. 2 is a schematic structural diagram of an atomization assembly according to this application, FIG. 3 is a schematic partial exploded structural view of the atomization assembly shown in FIG. 2, FIG. 4 is a schematic structural diagram of an atomization core shown in FIG. 2, and FIG. 5 is a schematic structural diagram of a dense base shown in FIG. 4.

The atomization assembly 1 includes a housing 10, a heating seat 11, and an atomization core 12. The housing 10 includes a liquid storage chamber 13, an air outlet channel 14, and an accommodating chamber 15. The liquid storage chamber 13 surrounds the air outlet channel 14, and the heating seat 11 is located inside the accommodating chamber 15. The housing 10 and the heating seat 11 together form a housing assembly. The liquid storage chamber 13 is configured to store a substance. The air outlet channel 14 is substantially parallel to the central axis of the atomization assembly 1, and one end of the air outlet channel 14 extends to the end surface of the housing 10 to form an inhalation opening 16. A user inhales through the inhalation opening 16. In an implementation, the entire the air outlet channel 14 is parallel to the central axis of the atomization assembly 1. The atomization core 12 is located inside the heating seat 11, and the atomization core 12 is configured to atomize the substance. The atomization surface of the atomization core 12 is arranged facing toward the sidewall of the housing 10. That is, the atomization surface of the atomization core 12 is exposed to an atomization chamber 17 and is substantially parallel to the air outlet channel 14. The atomization surface of the atomization core 12 cooperates with the heating seat 11 to form the atomization chamber 17. The atomization chamber 17 communicates with the air outlet channel 14. That is, the aerosol atomized by the atomization core 12 is inside the atomization chamber 17 and arrives at the inhalation opening 16 through the air outlet channel 14 to be inhaled by a user. An angle between the atomization surface of the atomization core 12 and the central axis of the atomization assembly 1 is less than 30°. In an implementation, the atomization surface of the atomization core 12 is parallel to the central axis of the atomization assembly 1.

The entire air outlet channel 14 is arranged parallel to the central axis of the atomization assembly 1, and one end of the air outlet channel 14 extends to the end surface of the housing 10 to form the inhalation opening 16. The atomization surface of the atomization core 12 is parallel to the central axis of the atomization assembly 1. The atomization chamber 17 formed by the atomization surface of the atomization core 12 and the heating seat 11 in cooperation communicates with the air outlet channel 14. The aerosol atomized by the atomization core 12 flows straightly from the atomization chamber 17 to the inhalation opening 16 to be inhaled by a user. In this way, the aerosol is prevented from passing through a detour, and the contact between the aerosol and the wall surface is reduced, thereby reducing the amount of formed condensate. In addition, the length of the air passage is reduced, making the temperature of the aerosol arriving at the mouth of a user appropriate, which is beneficial to improving the taste.

In an implementation, the atomization surface of the atomization core 12 is coplanar or tangent to a side surface of the air outlet channel 14, to further allow the aerosol to flow along a straight line to the inhalation opening 16. When the cross section of the air outlet channel 14 is circular, the atomization surface of the atomization core 12 is tangent to the side surface of the air outlet channel 14. When the cross section of the air outlet channel 14 is square, the atomization surface of the atomization core 12 is coplanar with a part of the side surface of the air outlet channel 14. Specifically, the distance between the atomization surface of the atomization core 12 and the axis of the air outlet channel 14 is a first value, the radius of the air outlet channel 14 (when the cross section of the air outlet channel 14 is circular) is a second value, and the first value is the same as the second value, to enable the aerosol to flow out along a straight channel. In other implementations, the first value may be greater than or is less than the second value.

In this embodiment, referring to FIG. 4 and FIG. 5, the atomization core 12 includes a dense base 121 and a heating film 122. The dense base 121 includes a first surface 1211 and a second surface 1212 opposite to the first surface 1211. The dense base 121is provided with a plurality of first micropores 1213. The first micropores 1213 are through holes running through the first surface 1211 and the second surface 1212. The heating film 122 is located on the first surface 1211. The surface of the dense base 121 on which the heating film 122 is arranged is the atomization surface. That is, the first surface 1211 of the dense base 121 is the atomization surface, and the second surface 1212 of the dense base 121 is a liquid-absorbing surface. That is, the dense base 121 includes the liquid-absorbing surface and the atomization surface opposite to the liquid-absorbing surface, and the heating film 122 is located on the atomization surface. The first micropores 1213 are through holes running through the liquid-absorbing surface and the atomization surface. The first micropores 1213 are configured to guide the substance from the liquid-absorbing surface to the atomization surface through the capillary force. The material of the dense base 121 is a dense ceramic or glass. When the material of dense base 121 is glass, the glass is borosilicate glass, quartz glass, or photosensitive lithium aluminosilicate glass.

The heating film 122 includes a plurality of second micropores 1221 having a one-to-one correspondence with and communicating with the plurality of first micropores 1213. The resistance of the heating film 122 of the atomization core 12 at normal temperature (20°C to 25°C) ranges from 0.5 ohms to 2 ohms. It may be understood that the dense base 121 plays a structural support role, and the heating film 122 in the atomization core 12 is electrically connected to the power supply assembly 2. The power of the electronic atomization device ranges from 6 watts to 8.5 watts, the voltage of the battery ranges 2.5 volts to 4.4 volts.

In this application, the dense base 121 is provided with the plurality of first micropores 1213 with the capillary force, to make the magnitude of the porosity of the atomization core 12 accurately controllable, thereby improving the consistency of products. That is to say, in mass production, the porosity of the dense base 121 in the atomization core 12 is basically consistent, and the thickness of the heating film 122 formed on the dense base 121 is even, for the electronic atomization device delivered in different batches to have the consistent atomization effect.

In another embodiment, the atomization core 12 may also be a sheet-shaped porous ceramic atomization core, which is specifically designed according to requirements.

The heating seat 11 includes a first sub-heating seat 111 and a second sub-heating seat 112. The first sub-heating seat 111 and the second sub-heating seat 112 cooperate with each other to clamp the atomization core 12, to fix the atomization core 12. Specifically, the first sub-heating seat 111 and the second sub-heating seat 112 clamp the two surfaces of the atomization core 12 in the direction perpendicular to the axial direction of the atomization assembly 1, to protect the atomization core 12 while fixing the atomization core 12 in the direction perpendicular to the atomization core 12, thereby improving the impact resistance capability of the atomization core 12 and preventing the atomization core 12 from breaking. In an implementation, the first sub-heating seat 111 and the second sub-heating seat 112 include a protrusion and a hook that correspond to each other and that are arranged in cooperation. The first sub-heating seat 111 and the second sub-heating seat 112 are snap-connected by the protrusion and the hook. In another implementation, the first sub-heating seat 111 and the second sub-heating seat 112 may also be connected in a manner such as interference fit or magnetic attraction.

Specifically, the first sub-heating seat 111 or the second sub-heating seat 112 is provided with a groove 1111. The groove 1111 communicates with the liquid storage chamber 13, and the atomization core 12 is arranged inside the groove 1111. In an implementation, the groove 1111 is provided on the first sub-heating seat 111, the atomization core 12 is arranged in the groove 1111, and the atomization surface of the atomization core 12 cooperates with the second sub-heating seat 112 to form the atomization chamber 17. In another implementation, a cavity (not shown) is formed on the second sub-heating seat 112. The cavity wall of the cavity includes a first sidewall and a second sidewall arranged opposite to each other. The first sidewall of the cavity is provided with the groove 1111, the atomization core 12 is arranged inside the groove 1111, and the atomization surface of the atomization core 12 cooperates with the second sidewall to form the atomization chamber 17. The second sidewall is located on the side of the first sidewall away from the first sub-heating seat 111. The manner of arranging the groove 1111 may be designed according to specific requirements, provided that the groove 1111 can have the atomization core 12 mounted therein and enable the atomization core 12 to come into contact with the substrate in the liquid storage chamber 13.

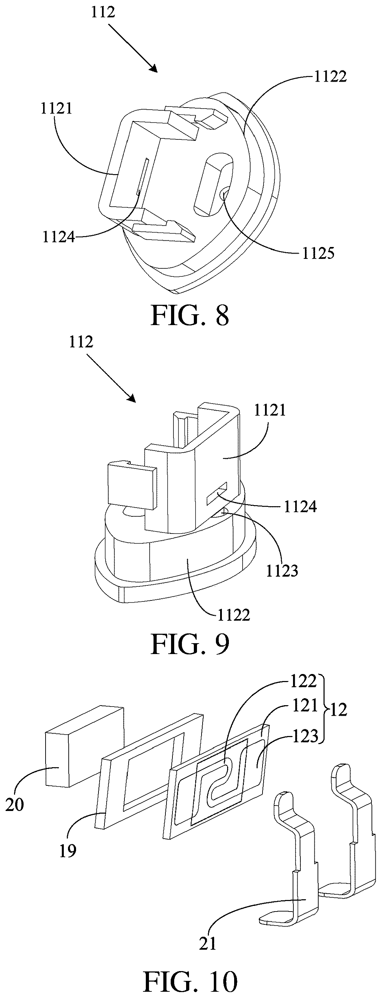

Referring to FIG. 6 to FIG. 9, FIG. 6 is a schematic structural diagram of a first sub-heating seat shown in FIG. 2, FIG. 7 is a schematic structural diagram of the first sub-heating seat shown in FIG. 6 from another perspective, FIG. 8 is a schematic structural diagram of a second sub-heating seat in FIG. 2, and FIG. 9 is a schematic structural diagram of the second sub-heating seat shown in FIG. 8 from another perspective.

Referring to FIG. 2 and FIG. 6 to FIG. 9, the first sub-heating seat 111 includes a top cover 1112 and a lower seat 1113, and the second sub-heating seat 112 includes an upper seat 1121 and a base 1122. The top cover 1112 of the first sub-heating seat 111 covers the base 1122 of the second sub-heating seat 112, and abuts against the upper seat 1121 of the second sub-heating seat 112, and the base 1122 of the second sub-heating seat 112 covers the top cover 1112 of the first sub-heating seat 111, and abuts against the lower seat 1113 of the first sub-heating seat 111, to facilitate assembly. It may be understood that, the top cover 1112 and the lower seat 1113 of the first sub-heating seat 111 may be integrally formed, or may be fixed together in a manner such as gluing, and the upper seat 1121 and base 1122 of the second sub-heating seat 112 may be integrally formed, or may be fixed together in a manner such as gluing, which may be specifically designed according to requirements.

A side surface of the lower seat 1113 of the first sub-heating seat 111 is provided with the groove 1111, and the atomization surface of the atomization core 12 cooperates with a side surface of the upper seat 1121 of the second sub-heating seat 112 to form the atomization chamber 17. The top cover 1112 of the first sub-heating seat 111 is provided with a liquid drain hole 1114 and a ventilation hole 1115. One end of the ventilation hole 1115 communicates with the atomization chamber 17, and the other end of the ventilation hole 1115 communicates with the air outlet channel 14. One end of the liquid drain hole 1114 communicates with the liquid storage chamber 13. The lower seat 1113 of the first sub-heating seat 111 is provided with a liquid drain channel 1116. One end of the liquid drain channel 1116 communicates with the other end of the liquid drain hole 1114, and the other end of the liquid drain channel 1116 communicates with the groove 1111. The base 1122 of the second sub-heating seat 112 is provided with an air inlet hole 1123, and the air inlet hole 1123 communicates with the atomization chamber 17. Specifically, external air enters the atomization chamber 17 through the air inlet hole 1123, carries the aerosol inside the atomization chamber 17, enters the air outlet channel 14 through the ventilation hole 1115, and further, reaches the inhalation opening 16 to be inhaled by a user. The liquid drain channel 1116 and the liquid drain hole 1114 may be a whole (when the liquid drain channel and the liquid drain hole have the same shape and the same pore size), or may be two separate parts (when the liquid drain channel and the liquid drain hole have different shapes). The liquid drain channel 1116 cooperates with the liquid drain hole 1114 to form a fluid channel.

In this embodiment, the liquid drain channel 1116 runs through the lower seat 1113 of the first sub-heating seat 111. That is, the liquid drain channel 1116 is a through hole running through the lower seat 1113. The bottom wall of the groove 1111 is provided with a liquid inlet hole 1117, for the groove 1111 to communicate with the liquid drain channel 1116. It may be understood that the structure of the liquid drain channel 1116 may be designed according to requirements provided that the groove 1111 can communicate with the liquid inlet hole 1117, and further communicate with the liquid storage chamber 13.

Referring to FIG. 8 and FIG. 9, the base 1122 of the second sub-heating seat 112 is provided with an air inlet hole 1123, and the upper seat 1121 of the second sub-heating seat 112 is provided with a through hole 1124 running through the upper seat 1121. The axis of the through hole 1124 is perpendicular to the height direction of the upper seat 1121, and the through hole 1124 communicates with the atomization chamber 17. The end part of the base 1122 forms a step structure (not shown), the end part of the housing 10 abuts against the step surface of the step structure, and the base 1122 blocks the end part of the housing 10. There is a gap between the upper seat 1121 and the housing 10, the air inlet hole 1123 is exposed to the gap, and the through hole 1124 communicates with the air inlet hole 1123 through the gap. It may be understood that, in another implementation, the base 1122 of the second sub-heating seat 112 is provided with an air inlet hole 1123, the air inlet hole 1123 is a through hole running through the base 1122, and the axis of the air inlet hole 1123 is parallel to the central axis of the atomization assembly 1. The manner of arranging the air inlet hole 1123 may be designed according to requirements provided that the external air can communicate with the atomization chamber 17.

Referring to FIG. 2, FIG. 3, and FIG. 6 to FIG. 9, the atomization assembly 1 further includes an isolation plug 18. The isolation plug 18 is partially arranged inside the liquid drain channel 1116 and/or the liquid drain hole 1114. When the isolation plug 18 is at a first position, the liquid storage chamber 13 does not communicate with the groove 1111. When the isolation plug 18 is at a second position, the liquid storage chamber 13 communicates with the groove 1111. That is, when the isolation plug 18 is at the first position, the isolation plug 18 blocks the liquid inlet hole 1117 on the bottom wall of the groove 1111 or blocks the liquid drain hole 1114, to prevent the liquid storage chamber 13 from communicating with the groove 1111. When the isolation plug 18 is at the second position, the isolation plug 18 does not block the liquid drain hole 1114 and the liquid inlet hole 1117 on the bottom wall of the groove 1111, to enable the liquid storage chamber 13 to communicate with the groove 1111.

The isolation plug 18 includes a sealing part 181 and a pull rod 182. The sealing part 181 is arranged inside the liquid drain channel 1116 and/or the liquid drain hole 1114, and the shape of the sealing part 181 matches the shape of the liquid drain channel 1116 and/or the liquid drain hole 1114. The pull rod 182 is arranged at the end of the sealing part 181 away from the liquid storage chamber 13. The base 1122 of the second sub-heating seat 112 is provided with a connection hole 1125, and the connection hole 1125 is provided corresponding to the liquid drain channel 1116. The pull rod 182 is arranged inside the connection hole 1125 to connect to the sealing part 181, and the end part of the pull rod 182 extends out of the side of the housing 10 facing away from the inhalation opening 16. The material of the sealing part 181 is silica gel, and the material of the pull rod 182 is silica gel, plastic, wood, or the like. The manner of the connection between sealing part 181 and the pull rod 182 may be designed according to requirements, for example, be integrally formed.

By arranging the isolation plug 18, the isolation plug 18 blocks the liquid inlet hole 1117 during delivery, for the isolation plug 18 to be at the first position, which reduces the risk of liquid leakage during transportation and storage. In use, the isolation plug 18 is pulled to the bottom to be located at the second position, for the substance to enter the groove 1111. After the isolation plug 18 is pulled to the bottom to be located at the second position, the pull rod 182 may also be cut off (a user may pull off the pull rod 182 and separate the pull rod 182 from the sealing part 181), to facilitate the electrical connection between the atomization assembly 1 and the power supply assembly 2.

It may be understood that, when the distance between the top surface of the sealing part 181 and the bottom wall of the liquid storage chamber 13 is the first value, the distance between the end of the liquid inlet hole 117 close to the liquid storage chamber 13 and the bottom wall of the liquid storage chamber 13 is the second value, and the first value is greater than the second value, if the length of the sealing part 181 is less than the height of the liquid inlet hole 1117, the substance may enter from the top surface of the sealing part 181 into the liquid inlet hole 1117 to flow the side of the atomization core 12 close to the atomization chamber 17, then flow from the side of the atomization core 12 close to the atomization chamber 17 to the other side of the atomization core 12, and further leak out from the liquid drain channel 1116. That is, if the length of sealing part 181 is less than the height of the liquid inlet hole 1117, there is a risk of liquid leakage. Therefore, the length of the sealing part 181 is set to be not less than the height of the liquid inlet hole 1117. The length of sealing part 181 is the distance between the top surface thereof and the bottom surface thereof along the length direction of atomization assembly 1. The height of the liquid inlet hole 1117 is the distance between the end of the liquid inlet hole 1117 close to the liquid storage chamber 13 and the end of the liquid inlet hole 1117 away from the liquid storage chamber 13.

Referring to FIG. 3, the atomization assembly 1 further includes a sealing member 19. The sealing member 19 is arranged between the atomization core 12 and the bottom wall of the groove 1111. The middle part of the sealing member 19 is provided with a communication hole 191, to expose at least a part of the atomization core 12, for the atomization core 12 to come into contact with the substance. Specifically, the communication hole 191 exposes at least some of the plurality of first micropores 1213 on the dense base 121 of the atomization core 12. The first micropores 1213 communicate with the liquid storage chamber 13 through the communication hole 191 and the groove 1111. The first micropores 1213 guide the substance from the liquid-absorbing surface to the atomization surface, for the substance to be atomized by the heating film 122 on the atomization surface. A sealing member 19 is arranged between the atomization core 12 and the bottom wall of the groove 1111, to buffer the atomization core 12, thereby improving the impact resistance capability of the atomization core 12.

The atomization assembly 1 further includes a liquid guiding element 20. The liquid guiding element 20 is arranged between the atomization core 12 and the bottom wall of the groove 1111. The liquid guiding element 20 is a liquid guiding material such as a liquid guiding cotton or a porous ceramic. The liquid guiding element 20 can be arranged to reduce uneven liquid absorption by the atomization core 12 when the liquid level of the substance is lower than the top of the liquid inlet hole 1117. The liquid guiding element 20 can absorb the liquid at the bottom of the liquid drain channel 1116 and distribute the supplied liquid evenly to the liquid-absorbing surface of the atomization core 12, which is beneficial to the consistency of the taste. The liquid guiding element 20 is arranged on the side of the sealing member 19 away from the atomization core 12.

Referring to FIG. 10, FIG. 10 is a partially enlarged view of some elements of the atomization assembly shown in according to FIG. 3.

The atomization core 12 further includes electrodes 123. The electrodes 123 are arranged on the atomization surface, and the heating film 122 is electrically connected to the electrodes 123. The atomization assembly 1 further includes an elastic piece 21, for example, a metal elastic piece. One end of the elastic piece 21 is connected to the electrodes 123, and the other end of the elastic piece 21 is connected to the power supply assembly 2. Specifically, the base 1122 of the second sub-heating seat 112 is arranged with a mounting hole (not shown), and the other end of the elastic piece 21 is arranged inside the mounting hole and exposed to the end part of the atomization assembly 1 to be electrically connected to the power supply assembly 2. In an implementation, the elastic piece 21 is a metal piece, and the elastic piece 21 is embedded into the second sub-heating seat 112 through injection molding, to facilitate assembly. Specifically, one end of the elastic piece 21 extends out of the second sub-heating seat 112 and is bent to form an elastic contact end for abutting against the electrodes 123. The other end of the elastic piece 21 is vertically bent to form a contact end that is parallel to the bottom surface of the second sub-heating seat 112 and that is exposed on the bottom surface of the second sub-heating seat 112 for abutting against an electrode pin/a connector pin of the power supply assembly 2. It may be understood that, the manner in which the elastic piece 21 is fixed to the second sub-heating seat 112 may be designed according to requirements, which is not limited in this application.

The material of the dense base 121 of the atomization core 12 being glass is described below in detail.

The thickness of the dense base 121 ranges from 0.1 mm to 1 mm. When the thickness of the dense base 121 is greater than 1 mm, the liquid supply demand cannot be satisfied, resulting in a reduced amount of the aerosol, a large amount of heat loss, and high costs of arranging the first micropores 1213. When the thickness of the dense base 121 is less than 0.1 mm, the strength of the dense base 121 cannot be guaranteed, which is not conducive to improvement in the performance of the electronic atomization device. The thickness of the dense base 121 ranges from 0.2 mm to 0.5 mm. The pore size of the first micropore 1213 on the dense base 121 ranges from 1 micrometer to 100 micrometers. When the pore size of the first micropore 1213 is less than 1 micrometer, the liquid supply demand cannot be satisfied, resulting in a reduced amount of the aerosol. When the pore size of the first micropore 1213 is greater than 100 micrometers, the substance easily flows out of the first micropore 1213 to the first surface 1211 to cause liquid leakage, resulting in the decreased atomization efficiency. The pore size of the first micropore 1213 ranges from 20 micrometers to 50 micrometers. It may be understood that the thickness of the dense base 121 and the pore size of the first micropore 1213 are selected according to actual requirements.

The ratio of the thickness of the dense base 121 to the pore size of first micropore 1213 ranges from 20:1 to 3:1. When the ratio of the thickness of the dense base 121 to the pore size of the first micropore 1213 is greater than 20:1, it is difficult for the substance supplied through the capillary force of the first micropores 1213 to satisfy the atomization demand amount of the atomization core 12, easily resulting in dry heating and a reduced amount of the aerosol generated through single atomization. When the ratio of the thickness of the dense base 121 to the pore size of the first micropore 1213 is less than 3:1, the substance may easily flow out from the first micropores 1213 to the first surface 1211 to cause the waste of the substance, resulting in the decreased atomization efficiency and further a reduced total amount of the aerosol.

The ratio of the distance between the centers of two adjacent first micropores 1213 to the pore size of the first micropore 1213 ranges from 3:1 to 1.5:1, for the strength of the dense base 121 to be improved as much as possible while causing the first micropores 1213 on the dense base 121 to meet the liquid supplying capability. The ratio of the distance between the centers of two adjacent first micropores 1213 to the pore size of the first micropore 1213 ranges from 3:1 to 2:1.

In a specific embodiment, the ratio of the thickness of the dense base 121 to the pore size of first micropore 1213 ranges from 15:1 to 5:1, and the ratio of the distance between the centers of two adjacent first micropores 1213 to the pore size of the first micropore 1213 ranges from 3:1 to 2.5:1.

The foregoing descriptions are merely some embodiments of this application, and the protection scope of this application is not limited thereto. All equivalent apparatus or process changes made according to the content of this specification and accompanying drawings in this application or by directly or indirectly applying this application in other related technical fields shall fall within the protection scope of this application.

## Claims

1. An atomization assembly, comprising:
a housing assembly, comprising a liquid storage chamber, an air outlet channel, and an atomization chamber, wherein the liquid storage chamber is configured to store a substance; and
an atomization core, configured to atomize the substance to generate an aerosol, wherein an atomization surface of the atomization core is exposed to the atomization chamber and is substantially parallel to the air outlet channel, and the atomization chamber communicates with the atomization surface of the atomization core and the air outlet channel.

2. The atomization assembly of claim 1, wherein the housing assembly comprises:
a housing; and
a heating seat, located inside the housing, and cooperating with the housing to form the liquid storage chamber, wherein the atomization core is located inside the heating seat.

3. The atomization assembly of claim 1, wherein the atomization surface of the atomization core is substantially parallel to the central axis of the atomization assembly; and the air outlet channel is substantially parallel to the central axis of the atomization assembly, and an inhalation opening of a housing communicates with the atomization chamber through the air outlet channel.

4. The atomization assembly of claim 1, wherein a heating seat comprises a fluid channel; the fluid channel communicates with the liquid storage chamber, and the sidewall of the fluid channel is provided with a liquid inlet hole, to enable the atomization core to communicate with the liquid storage chamber; and
the atomization assembly further comprises an isolation plug, and the isolation plug comprises a sealing part and a pull rod; and
the sealing part is arranged inside the fluid channel, the pull rod is arranged at the end of the sealing part away from the liquid storage chamber, and the length of the sealing part is not less than the length of the fluid channel.

5. The atomization assembly of claim 1, wherein the heating seat comprises a first sub-heating seat and a second sub-heating seat that are fixedly connected, and the first sub-heating seat and the second sub-heating seat cooperate with each other to clamp the atomization core.

6. The atomization assembly of claim 5, wherein one of the first sub-heating seat and the second sub-heating seat comprises a protrusion and the other of the first sub-heating seat and the second sub-heating seat comprises a hook, and the protrusion is arranged in cooperation with the hook; and
the first sub-heating seat and the second sub-heating seat are snap-connected by the protrusion and the hook.

7. The atomization assembly of claim 5, wherein the first sub-heating seat or the second sub-heating seat is provided with a groove; and the groove communicates with the liquid storage chamber, and the atomization core is arranged inside the groove.

8. The atomization assembly of claim 7, wherein the atomization assembly further comprises a sealing member, and the sealing member is arranged between the atomization core and the bottom wall of the groove; and
the middle part of the sealing member is provided with a communication hole, to expose at least a part of the atomization core.

9. The atomization assembly of claim 7, wherein the atomization assembly further comprises a liquid guiding element, and the liquid guiding element is arranged between the atomization core and the bottom wall of the groove.

10. The atomization assembly of claim 9, wherein the liquid guiding element is a liquid guiding cotton or a porous ceramic.

11. The atomization assembly of claim 7, wherein the first sub-heating seat comprises a top cover and a lower seat, and the second sub-heating seat comprises an upper seat and a base;
the top cover of the first sub-heating seat covers the base of the second sub-heating seat and abuts against the upper seat of the second sub-heating seat; and
the base of the second sub-heating seat covers the top cover of the first sub-heating seat and abuts against the lower seat of the first sub-heating seat, and a side surface of the lower seat of the first sub-heating seat is provided with the groove.

12. The atomization assembly of claim 11, wherein the top cover of the first sub-heating seat is provided with a liquid drain hole and a ventilation hole; one end of the ventilation hole communicates with the atomization chamber, and the other end of the ventilation hole communicates with the air outlet channel;
one end of the liquid drain hole communicates with the liquid storage chamber;
the lower seat of the first sub-heating seat is arranged with a liquid drain channel, one end of the liquid drain channel communicates with the other end of the liquid drain hole, and the other end of the liquid drain channel communicates with the groove; and
the atomization surface of the atomization core cooperates with a side surface of the upper seat of the second sub-heating seat to form the atomization chamber; and the base of the second sub-heating seat is provided with an air inlet hole, and the air inlet hole communicates with the atomization chamber.

13. The atomization assembly of claim 12, wherein the liquid drain channel runs through the lower seat of the first sub-heating seat; the bottom wall of the groove is provided with a liquid inlet hole, to enable the groove to communicate with the liquid drain channel; and
the atomization assembly further comprises an isolation plug, and the isolation plug is partially arranged inside the liquid drain channel and/or the liquid drain hole; and when the isolation plug is located at a first position, the liquid storage chamber does not communicate with the groove, and when the isolation plug is located at a second position, the liquid storage chamber communicates with the groove.

14. The atomization assembly of claim 13, wherein the isolation plug comprises a sealing part and a pull rod; the sealing part is arranged inside the liquid drain channel and/or the liquid drain hole, and the pull rod is arranged at the end of the sealing part away from the liquid storage chamber; the base of the second sub-heating seat is provided with a connection hole, and the connection hole is provided corresponding to the liquid drain channel; the pull rod is arranged inside the connection hole, to connect to the sealing part; and the end part of the pull rod extends out of the side of the housing facing away from the inhalation opening.

15. The atomization assembly of claim 14, wherein the length of the sealing part is not less than the height of the liquid drain hole.

16. The atomization assembly of claim 11, wherein the atomization core comprises a dense base and a heating film;
the dense base comprises a liquid-absorbing surface and the atomization surface opposite to the liquid-absorbing surface, the dense base is provided with a plurality of first micropores, the first micropores are through holes running through the liquid-absorbing surface and the atomization surface, and the first micropores are configured to guide the substance to the atomization surface; and the heating film is arranged on the atomization surface.

17. The atomization assembly of claim 16, wherein the atomization core further comprises electrodes, the electrodes are arranged on the atomization surface, and the heating film is electrically connected to the electrodes;
the atomization assembly further comprises an elastic piece, one end of the elastic piece is connected to the electrodes, and the other end of the elastic piece is connected to the power supply assembly; and
the base of the second sub-heating seat is provided with a mounting hole, and the other end of the elastic piece is arranged inside the mounting hole; and
the elastic piece and the second sub-heating seat are injection molded.

18. The atomization assembly of claim 16, wherein the dense base is glass, and the glass is borosilicate glass, quartz glass, or photosensitive lithium aluminosilicate glass.

19. The atomization assembly of claim 16, wherein the thickness of the dense base ranges from 0.1 mm to 1 mm; and the pore size of the first micropore ranges from 1 µm to 100 µm.

20. The atomization assembly of claim 11, wherein the first sub-heating seat and the second sub-heating seat are both integrally formed.

21. An electronic atomization device, comprising an atomization assembly and a power supply assembly, wherein the atomization assembly is the atomization assembly of any one of claims 1 to 20, and the power supply assembly is configured to control the atomization assembly to work.
